# EUROPEAN PATENT SPECIFICATION

(11) **EP 2 868 266 B1**
(45) Date of publication and mention of the grant of the patent: **10.10.2018**
(21) Application number: 14189004.6
(22) Date of filing: 15.10.2014
(51) Int. Cl.: A61B 5/00, A61B 8/08

(54) **Subject-information acquiring apparatus**
Vorrichtung zur Gewinnung von Objektinformationen
Appareil d'acquisition d'informations sur un objet

(30) Priority: 31.10.2013 JP 2013227236; 07.10.2014 JP 2014206743
(43) Date of publication of application: 06.05.2015
(73) Proprietor: Canon Kabushiki Kaisha, Tokyo 146-8501 (JP)
(72) Inventor: Kyono, Hisako, Tokyo, Tokyo 146-8501 (JP); Furukawa, Yukio, Tokyo, Tokyo 146-8501 (JP); Motoki, Yohei, Tokyo, Tokyo 146-8501 (JP); Nishihara, Hiroshi, Tokyo, Tokyo 146-8501 (JP); Nagao, Daisuke, Tokyo, Tokyo 146-8501 (JP)
(74) Representative: TBK

(56) References cited:
- WO-A1-02/091927
- WO-A1-2012/108172
- WO-A1-2013/049629
- WO-A2-2004/006755

## Description

### BACKGROUND OF THE INVENTION

### Field of the Invention

The present invention relates to a subject-information acquiring apparatus that acquires information about a subject, such as a living body. The present invention particularly relates to an apparatus that uses a photoacoustic effect.

### Description of the Related Art

A photoacoustic effect refers to a phenomenon in which when a portion to be examined (hereinafter referred to as an examined portion) is irradiated with pulsed light generated from a light source, acoustic waves are generated inside the examined portion by absorption of light. There is a technique called photoacoustic tomography (PAT) that uses the photoacoustic effect for imaging of internal tissue from which acoustic waves are generated. The photoacoustic tomography is proposed as a technique for imaging of physiological information, that is, functional information about a living body or the like.

The level of resolution achieved in the photoacoustic tomography depends on the arrangement of acoustic wave detectors. It is known that a high resolution can be achieved when a plurality of acoustic wave detectors are arranged such that the directions of maximum sensitivities of their reception directivities intersect each other.

In the device disclosed in U.S. Patent Application Publication No. 2011/0306865, acoustic wave detectors are arranged in the above-described manner to receive acoustic waves. In this device, a holder is provided between an examined portion and the acoustic wave detectors to hold the examined portion.

The holder in the device disclosed in U.S. Patent Application Publication No. 2011/0306865 has a fixed shape. Therefore, if each area to be examined has a different shape, for example, if the examined portion of each subject has a different shape, the holder may not fit the shape of the examined portion. If the holder does not fit the shape of the examined portion, it may be difficult to properly hold the examined portion. As a result, it may not be possible to acquire accurate information about the inside of the examined portion.

Prior art which is related to this field of technology can be found e.g. in document WO 2012/108172 A1 disclosing acoustic-wave acquisition apparatus, in document WO 2004/006755 A2 disclosing support bra for ultrasonic breast scanner, in document WO 2013/049629 A1 disclosing interfacing systems, devices, and methods for optical imaging, and in document WO 02/091927 A1 disclosing ultrasonic breast examination system.

### SUMMARY OF THE INVENTION

The present invention in its aspect provides a subject-information acquiring apparatus as specified in claims
1 to 10.

Further features of the present invention will become apparent from the following description of exemplary embodiments with reference to the attached drawings.

### BRIEF DESCRIPTION OF THE DRAWINGS

Fig. 1 is a block diagram of a first embodiment.
Fig. 2 is a schematic diagram of Example 1.
Fig. 3 is a schematic diagram of Example 2.
Fig. 4 is a block diagram of a second embodiment.
Figs. 5A and 5B are schematic diagrams of Example 3.
Fig. 6 is a flowchart of Example 3.
Fig. 7 is a block diagram of Example 4.
Figs. 8A and 8B are schematic diagrams of Example 4.
Fig. 9 is a flowchart of Example 4.
Fig. 10 is a block diagram of Example 5.
Figs. 11A and 11B are schematic diagrams of Example 5.
Fig. 12 is a flowchart of Example 5.
Fig. 13 is a block diagram of Example 6.
Figs. 14A and 14B are operation diagrams of Example 6.
Fig. 15 is a block diagram of Example 7.
Fig. 16 illustrates a scanning path.
Fig. 17 is a block diagram of Example 8.
Figs. 18A and 18B are schematic diagrams of Example 8.
Fig. 19 is a flowchart of Example 8.

### DESCRIPTION OF THE EMBODIMENTS

Embodiments to which the present invention is applied will now be described in detail with reference to the drawings.

### First Embodiment

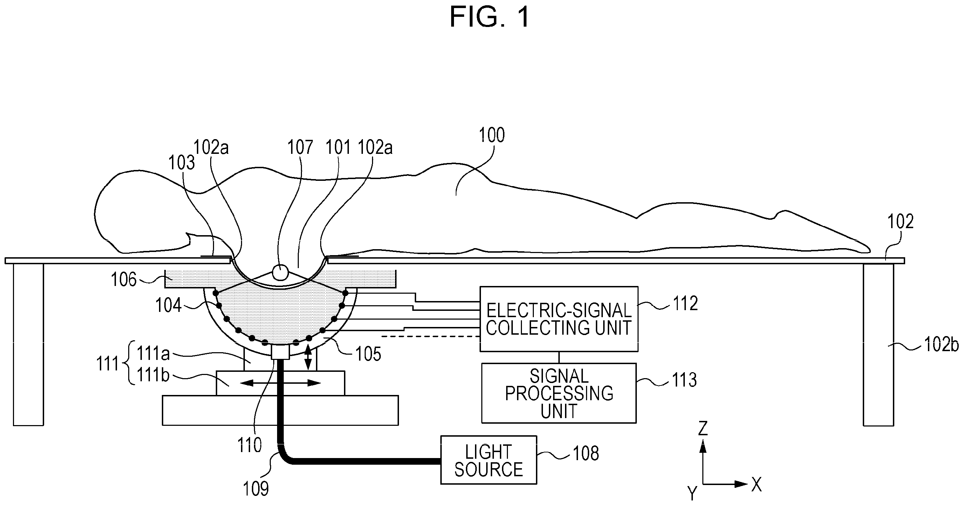

As illustrated in Fig. 1, a subject-information acquiring apparatus according to a first embodiment of the present invention includes a light source 108, a holder 103 that holds a portion to be examined (hereinafter referred to as an examined portion) 101 which is part of a subject 100, and a bed 102 that supports the subject 100. The subject-information acquiring apparatus also includes a plurality of acoustic wave detectors 104 that detect acoustic waves and output electric signals. The acoustic waves are generated when the examined portion 101 held by the holder 103 is irradiated with light emitted from the light source 108. The subject-information acquiring apparatus further includes a supporting member 105 that supports the plurality of acoustic wave detectors 104. The supporting member 105 supports the plurality of acoustic wave detectors 104 such that the direction of maximum reception sensitivity of at least some of the plurality of acoustic wave detectors 104 differs from the direction of maximum reception sensitivity of other acoustic wave detectors 104 different from the at least some of the plurality of acoustic wave detectors 104. Also, the supporting member 105 supports the plurality of acoustic wave detectors 104 such that the direction of maximum reception sensitivity of the at least some of the plurality of acoustic wave detectors 104 and the direction of maximum reception sensitivity of other acoustic wave detectors 104 different from the at least some of the plurality of acoustic wave detectors 104 are directed toward a specific region. The subject-information acquiring apparatus further includes a signal processing unit 113 that acquires information about the inside of the examined portion 101 on the basis of electric signals output by the acoustic wave detectors 104. The holder 103 can be changed in shape to fit the shape of the examined portion 101. This allows accurate acquisition of information about the inside of the examined portion 101. This will be described below.

In a subject-information acquiring apparatus using the photoacoustic effect, it is desirable that an examined portion be in an appropriate shape during information acquisition (which hereinafter may be referred to as during measurement). This is because the conditions of light irradiation and the conditions of acoustic wave reception (or the conditions of acoustic wave propagation) are dependent on the shape of the examined portion. This means that depending on the shape of the examined portion, a desired area may not be irradiated with light, or generated acoustic waves may be attenuated and may be unable to be received. Since the examined portion varying from person to person (or individual to individual) does not necessarily have a shape suitable for measurement, it is desirable that the examined portion be held by a holder and measured after being formed into an appropriate shape. However, if the holder has a fixed shape, the holder may be unable to support the examined portion varying from person to person (or from individual to individual), and the examined portion may not be formed into an appropriate shape depending on its size or hardness. Moreover, if the shape (including the size and hardness) of the holder having a fixed shape differs greatly from that of the examined portion, the propagation of acoustic waves may be interrupted because it is possible not only that the examined portion cannot be formed into an appropriate shape, but also that a gap may be created between the holder and the examined portion. In the subject-information acquiring apparatus of the present embodiment, as described above, the holder 103 can be changed in shape to fit the shape of the examined portion 101. Therefore, it is possible to solve the problems described above and accurately acquire information about the inside of the examined portion 101. For example, the expression "the holder 103 can be changed in shape to fit the shape of the examined portion 101" means, as detailed in concrete examples below, that a cup-shaped member having a shape that fits the shape of the examined portion 101 is selected from a plurality of cup-shaped members of different sizes and placed as the holder 103 in the bed 102, or that an elastic member is used as the holder 103. In other words, the subject-information acquiring apparatus of the present invention can change the shape of the holder 103 so that the holder 103 fits the shape of the examined portion 101.

The subject-information acquiring apparatus of the first embodiment will be further described with reference to Fig. 1.

As illustrated, the bed 102 on which the subject 100 lies face-down has an opening portion 102a for insertion of the examined portion 101 and legs 102b for maintaining the height of the bed 102.

The examined portion 101 is held by the holder 103. When a material having an acoustic impedance close to that of a human body is selected as a material for the holder 103, it is possible to suppress reflection of acoustic waves (which may hereinafter be referred to as ultrasonic waves) at the interface between the examined portion 101 and the holder 103. When the holder 103 is thin in thickness, it is possible to prevent reflection of ultrasonic waves from the holder 103 and reduce adverse noise. Therefore, the holder 103 having a small thickness may be used.

In the subject-information acquiring apparatus using the photoacoustic effect, the examined portion 101 is irradiated with light through the holder 103. Therefore, a material having a high light transmittance (preferably 90% or more) may be used for the holder 103. Examples of the material that satisfies the conditions described above include polymethylpentene and polyethylene terephthalate. The holder 103 can be changed in shape to fit the shape of the examined portion 101 of the subject 100. Concrete configurations will be described in Examples 1 and 2 below.

As described above, the acoustic wave detectors 104 are configured to detect generated acoustic waves and output electric signals. The acoustic wave detectors 104 that receive acoustic waves from the examined portion 101 may have a high sensitivity and a wide frequency band. Specifically, the acoustic wave detectors 104 made of lead zirconate titanate (PZT) or formed by capacitive micromachined ultrasonic transducers (CMUTs) may be used. However, the acoustic wave detectors 104 are not limited to particular ones, and may be of any type as long as they perform their functions.

The acoustic wave detectors 104 are supported by the supporting member 105. An acoustic matching liquid 106 needs to be a material that has an acoustic impedance close to that of a human body and does not significantly attenuate the ultrasonic waves. For example, water or oil may be used as the acoustic matching liquid 106.

The supporting member 105 is a container having a curvature in the surface that supports the acoustic wave detectors 104. The upper part of the container is structured such that a space defined by the bed 102, the holder 103, and the supporting member 105 can be filled with the acoustic matching liquid 106. If there is an air space between the examined portion 101 and the acoustic wave detectors 104, the detection of ultrasonic waves is interrupted because the ultrasonic waves are reflected at the interface due to a difference in acoustic impedance between the air and the examined portion 101. When the acoustic wave detectors 104 are arranged in the supporting member 105 filled with the acoustic matching liquid 106 as illustrated in Fig. 1, it is possible to reduce attenuation of ultrasonic waves. The acoustic wave detectors 104 are arranged in the surface of the supporting member 105, the surface being in contact with the acoustic matching liquid 106, such that the holder 103 is surrounded by the acoustic wave detectors 104. The receiving surfaces of the acoustic wave detectors 104 are positioned in the surface of the supporting member 105 such that the directions of maximum sensitivities of their reception directivities are directed toward (or preferably intersect at) a specific region (specifically a predetermined region of interest) of the examined portion 101. Thus, since the directions of maximum reception sensitivities of the acoustic wave detectors 104 are directed toward a specific region, photoacoustic waves generated from the specific region can be received with sensitivity higher than in the case where the directions of maximum reception sensitivities are parallel to each other. Thus, as compared to the case where the directions of maximum reception sensitivities are parallel to each other, the resolution of an image in the specific region can be increased.

The plurality of acoustic wave detectors 104 may be arranged in the surface of the supporting member 105 such that an angle formed by a first direction which is a direction of maximum reception sensitivity of at least some of the acoustic wave detectors 104 and a second direction which is a direction of maximum reception sensitivity of other acoustic wave detectors 104 different from the at least some of the acoustic wave detectors 104 is greater than 0 degrees and smaller than 180 degrees. The plurality of acoustic wave detectors 104 may be arranged in the surface of the supporting member 105 such that a first direction which is a direction of maximum reception sensitivity of at least some of the acoustic wave detectors 104 differs from a second direction which is a direction of maximum reception sensitivity of other acoustic wave detectors 104 different from the at least some of the acoustic wave detectors 104, and that acoustic waves from a specific region can be received with highest sensitivity by the acoustic wave detectors 104. When the subject-information acquiring apparatus includes the holder 103 as illustrated in Fig. 1, the plurality of acoustic wave detectors 104 may be arranged in the surface of the supporting member 105 such that a first direction which is a direction of maximum reception sensitivity of at least some of the acoustic wave detectors 104 differs from a second direction which is a direction of maximum reception sensitivity of other acoustic wave detectors 104 different from the at least some of the acoustic wave detectors 104, and that the first direction and the second direction are directed toward the holder 103. The supporting member 105 having a recessed portion may support the plurality of acoustic wave detectors 104 in the recessed surface of the supporting member 105 such that a first direction which is a direction of maximum reception sensitivity of at least some of the plurality of acoustic wave detectors 104 differs from a second direction which is a direction of maximum reception sensitivity of other acoustic wave detectors 104 different from the at least some of the acoustic wave detectors 104, and that the first direction and the second direction are directed toward the inside of the recessed portion. When the surface of the recessed portion is a spherical surface as illustrated in Fig. 1, the plurality of acoustic wave detectors 104 may be arranged in the spherical surface such that the first direction and the second direction are directed toward the inside of the spherical surface, so that the first direction and the second direction are directed toward the center of curvature of the spherical surface. In the present specification, the term "spherical surface" includes spherical surfaces other than a surface on a sphere. That is, the term "spherical surface" includes a spherical surface having an opening, such as a hemispherical surface. The term "spherical surface" also includes a spherical surface with unevenness and a surface on an ellipsoid, the surface being able to be regarded as a spherical surface (note that an ellipsoid is obtained by expanding an ellipse into a three-dimensional shape and its surface is a quadric surface). The surface of the recessed portion is not limited to a spherical surface, and may be a curved surface or a surface formed by combining a plurality of flat surfaces (preferably an angle formed by the flat surfaces is an obtuse angle). Also, the direction of maximum reception sensitivity described above is typically the direction of the normal to the receiving surface of the acoustic wave detector 104. This means that when the first direction and the second direction are directed toward the specific region (or the holder 103, the inside of the recessed portion, or the inside of the spherical surface), the receiving surfaces of the acoustic wave detectors 104 are directed toward the specific region (or the holder 103, the inside of the recessed portion, or the inside of the spherical surface).

When the acoustic wave detectors 104 are arranged as described above, a point at which the reception directivities of the acoustic wave detectors 104 intersect has the highest resolution. In the present embodiment, a region of high resolution in the vicinity of the highest resolution point is defined as a high-resolution region (specific region) 107. For example, the high-resolution region 107 may be a region having a resolution half the resolution at the highest resolution point.

The light source 108 generates pulsed light. Specifically, a TiS laser may be used as the light source 108, but the light source 108 is not limited to this. When the examined portion 101 is a living body, the pulse width of pulsed light from the light source 108 may be about 10 nanoseconds to 50 nanoseconds. The pulsed light may have a wavelength that allows propagation of light to the inside of the examined portion 101. Specifically, the wavelength of the pulsed light is in the 600 nm to 1100 nm range. A light transmitting portion 109 serves as a light guiding portion that transmits pulsed light generated by the light source 108. Specifically, a fiber bundle is used as the light transmitting portion 109. A light irradiation portion 110 (corresponding to an exit end) irradiates the holder 103 with light exiting from an exit portion of the light transmitting portion 109 and diffused by a diffusing plate (not shown).

A moving unit 111 that moves the supporting member 105 includes a Z-direction (vertical) moving mechanism 111a and an XY-direction (horizontal) moving mechanism 111b. The moving unit 111 moves the supporting member 105 relative to the holder 103. The moving unit 111 moves the supporting member 105 using a motor-driven xyz stage equipped with a stepping motor or the like. The moving unit 111 is not limited to this and may be of any type as long as it allows relative movement of the holder 103 and the supporting member 105.

An electric-signal collecting unit 112 collects a plurality of electric signals from the acoustic wave detectors 104 in a time sequence. A signal processing unit 113 amplifies analog electric signals output from the acoustic wave detectors 104, converts the amplified signals into digital signals, and acquires information about the inside of the examined portion 101.

A configuration of the holder 103 in the subject-information acquiring apparatus configured as described above will now be described using examples.

### (Example 1)

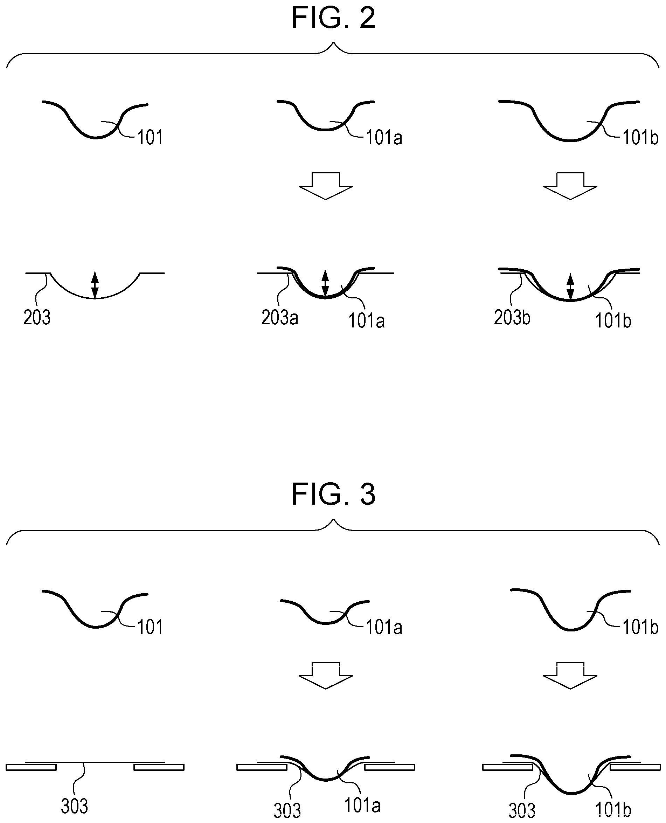

In the present example, the holder 103 is a cup-shaped member. Of a plurality of cup-shaped members of different sizes, a cup-shaped member having a size that fits the shape of the examined portion 101 is placed in the bed 102. Fig. 2 is a schematic diagram of the holder 103 to which the present embodiment is applicable.

In the present example, the holder 103 is a cup-shaped member (hereinafter described as a holding cup 203) and is placed in the opening portion 102a of the bed 102. The holding cup 203 may be secured to the opening portion 102a of the bed 102 by screwing, fitting, bonding, or any other method as long as the holding cup 203 can be secured to the opening portion 102a.

A plurality of holding cups 203 of different sizes are provided to accommodate various examined portions 101 of different (large and small) volumes. The holding cups 203 of different sizes mean the holding cups 203 of different volumes. For example, the volume of the holding cup 203 may be changed by varying the size in the depth direction (Z-direction in Fig. 1) while keeping the width constant, or by varying the size in the width direction while keeping the depth constant. The method for changing the volume of the holding cup 203 is not limited to this.

The holding cup 203 may be of a cylindrical shape, a rectangular parallelepiped shape, a bowl shape, or any other shape as long as the holding cup 203 can contact and hold the examined portion 101. Since the examined portion 101 is in a bell shape while the subject 100 is lying face-down, the holding cup 203 may have a bowl shape that fits the shape of the examined portion 101 and increases the area of contact between the examined portion 101 and the holding cup 203.

Fig. 2 illustrates an example in which the volume of the holding cup 203 is controlled by controlling the curvature of the holding cup 203 while keeping the depth of the holding cup 203 constant. In this case, when the examined portion 101 of the subject 100 is large (hereinafter, the examined portion 101 having a larger size will be described as a large examined portion 101b), the curvature of the holding cup 203 is increased to increase the volume of the holding cup 203 (hereinafter, the holding cup 203 having a large size will be described as a large holding cup 203b). When the examined portion 101 of the subject 100 is small (hereinafter, the examined portion 101 having a small size will be described as a small examined portion 101a), the curvature of the holding cup 203 is decreased (hereinafter, the holding cup 203 having a small size will be described as a small holding cup 203a). Thus, by selecting the volume of the holding cup 203 in accordance with the volume of the examined portion 101 of the subject 100, it is possible not only to hold the examined portion 101 in a desired shape, but also to increase the area of contact between the examined portion 101 and the holding cup 203 and hold the examined portion 101 without creating a gap between them. The same effect can be achieved by controlling the depth of the holding cup 203 while keeping the width constant, instead of controlling the curvature of the holding cup 203 while keeping the depth constant.

As described above, in the present example, even when the shape of the examined portion 101 varies for each subject 100, if the technician selects the holding cup 203 that fits the examined portion 101 of the subject 100, the examined portion 101 can be held in a desired shape by the holder 103. Then, when the examined portion 101 in this state is irradiated with light and the resulting acoustic waves are detected by the acoustic wave detectors 104, information about the inside of the examined portion 101 can be accurately acquired.

An additional effect will be described, which is achieved when the volume of the holding cup 203 is controlled by varying the curvature while keeping the depth constant. By varying the size of the holding cup 203 while keeping the depth constant, it is possible to always capture the image of the examined portion 101 in the high-resolution region 107 for the acoustic wave detectors 104 without adjusting the position of the high-resolution region 107 in the height direction. Therefore, the examined portion 101 which may vary in shape for each subject 100 can be scanned without causing the scanning range in the height direction to go off the high-resolution region 107. A reconstructed image having a high resolution can thus be obtained.

### (Example 2)

Unlike Example 1 in which the holding cup 203 selected from cup-shaped members of different sizes is used as the holder 103, an elastic member is used as the holder 103 in the present example. Fig. 3 is a schematic diagram of another holder 103 to which the present embodiment is applicable.

In the present example, a flat elastic sheet member 303 is placed as the holder 103 in the opening portion 102a of the bed 102. The sheet member 303 is preferably an elastic rubber member, and is more preferably made of a material having a low rubber hardness. For example, the rubber hardness may be 50 or less. The sheet member 303 may be strong enough to hold the examined portion 101 of the subject 100 without being broken.

As illustrated in Fig. 3, when holding the examined portion 101 of the subject 100, the sheet member 303 having elasticity deforms in accordance with the distribution of pressure applied from the examined portion 101 of the subject 100. When the sheet member 303 is made of a material having a low rubber hardness, it is possible to reduce the flexural rigidity of the sheet member 303. Thus, since no significant reactive force acts on the examined portion 101, it is possible to reduce discomfort and pain for the subject 100.

Also, when the sheet member 303 made of elastic rubber is used as the holder 103, the sheet member 303 can deform to follow the shape of the examined portion 101 which may vary in size and shape. It is thus possible to properly hold the examined portion 101.

The same effect can be achieved even when, for example, the holder 103 is bowl-shaped or cup-shaped. That is, the holder 103 of the present example is not necessarily limited to a flat sheet member.

In the present example, even when the examined portion 101 varies in size and shape, for example, for each subject 100, the examined portion 101 can be properly held by the holder 103 because the sheet member 303 deforms to follow the shape of the examined portion 101. Then, when the examined portion 101 in this state is irradiated with light and the resulting acoustic waves are detected by the acoustic wave detectors 104, information about the inside of the examined portion 101 can be accurately acquired.

An additional effect is that by using the sheet member 303 made of elastic rubber, it is possible to improve work efficiency of the technician, because there is no need to replace the holder 103 for each subject 100.

### Second Embodiment

A second embodiment of the present invention will be described on the basis of examples. In the second embodiment, the operation of the subject-information acquiring apparatus is controlled in accordance with the shape of the holder 103. First, the detection of the shape of the holder 103 common to the examples will be described with reference to the drawings. Note that overlapping description will be omitted.

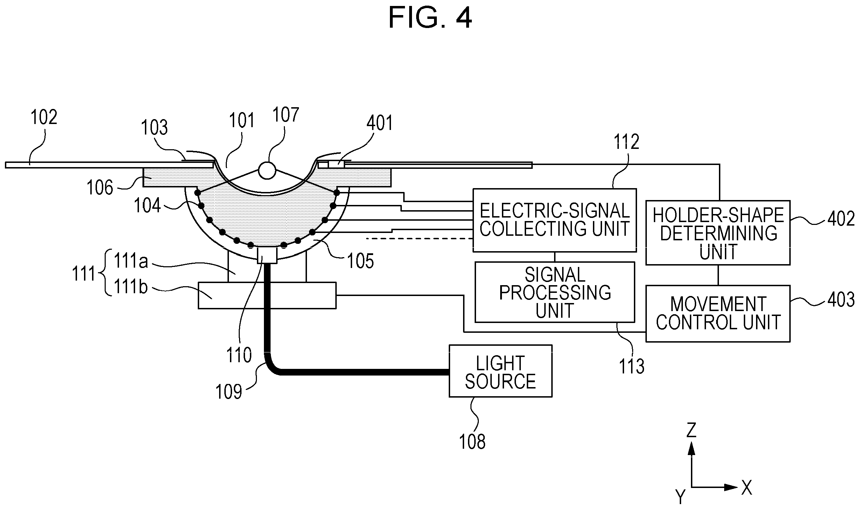

Referring to Fig. 4, a shape detecting unit 401 detects the shape of the holder 103. Here, the holding cup 203 is used as the holder 103. Specifically, the holding cup 203 is equipped with an integrated circuit (IC) chip, from which information is read by a reader. Instead of using the shape detecting unit 401 illustrated in Fig. 4, the subject-information acquiring apparatus may detect the size of the holding cup 203 input to an apparatus input unit (not shown) by the examiner. The method for detecting the size of the holding cup 203 is not limited to this, and any technique may be used as long as the size of the holding cup 203 can be detected by the subject-information acquiring apparatus.

When the sheet member 303 having elasticity is used as the holder 103, the shape information about the holder 103 can be acquired, for example, by using a camera. In this case, the shape information about the holder 103 may be input by the technician who views an image captured by the camera, or may be acquired by image processing. The shape information about the holder 103 may be acquired by using a three-dimensional measuring technique, such as a stereo method, on the basis of images captured from a plurality of directions, or may be acquired by using a contact probe, instead of a camera. The shape information about the holder 103 may be acquired from an image obtained from ultrasonic waves transmitted and received by the acoustic wave detectors 104. Then, the shape of the holder 103 may be detected by inputting the acquired shape information to the apparatus input unit (not shown).

A holder-shape determining unit 402 illustrated in Fig. 4 determines the shape of the holder 103 on the basis of the shape information about the holder 103 detected by the shape detecting unit 401 or the like.

On the basis of the following examples, a description will be given of how the operation of the subject-information acquiring apparatus is controlled in accordance with the shape of the holder 103 determined by the holder-shape determining unit 402.

### (Example 3)

The present example will be described with reference to Figs. 4 to 6. In the present example, the moving range of the supporting member 105 is controlled in accordance with the shape of the holder 103.

A movement control unit 403 controls the moving range of the moving unit 111 on the basis of information about the shape of the holder 103 determined by the holder-shape determining unit 402. When the shape of the holder 103 changes, a proper scanning range for acquiring information about the inside of the examined portion 101 also changes. Therefore, the scanning range is controlled to be appropriate for the shape of the holder 103.

In the present example, the holding cup 203 is used as the holder 103. The components common to those in the examples described above are given the same reference numerals and their detailed description will be omitted.

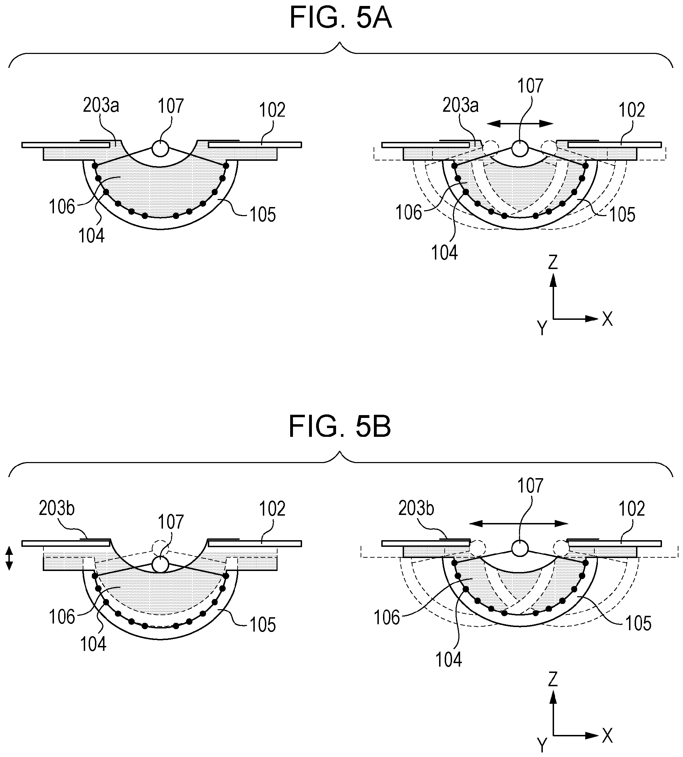

Fig. 5A illustrates a scanning range for the small holding cup 203a and Fig. 5B illustrates a scanning range for the large holding cup 203b. Although two types of sizes (large and small sizes) are described in the present example, the sizes are not limited to this.

When the high-resolution region 107 is moved for scanning the entire area of the holding cup 203, the scanning range for the large holding cup 203b is larger than that for the small holding cup 203a in scanning in the Z-direction and the XY-directions. Therefore, when the acoustic wave detectors 104 are moved for scanning the large holding cup 203b, if the large holding cup 203b is scanned in the scanning range for the small holding cup 203a, the scanning range of the high-resolution region 107 does not cover the entire range to be measured. That is, since the scanning range is narrow, the acoustic wave detectors 104 are moved over only part of the large holding cup 203b, so that information about only part of the inside of the examined portion 101 can be acquired. Conversely, when the acoustic wave detectors 104 are moved for scanning the small holding cup 203a, if the small holding cup 203a is scanned in the scanning range for the large holding cup 203b, the measurement time is increased because the scanning continues longer than necessary. By controlling the scanning range in accordance with the shape of the holder 103, the scanning range of the high-resolution region 107 can cover the entire range to be measured. This makes it possible to acquire a high-quality image of the range to be measured. That is, in accordance with the shape of the holder 103, the moving unit 111 controls the amount of displacement of the center position of the supporting member 105 with respect to the center position of the holder 103. This makes it possible to control the size of the moving range (scanning range) in the XY-directions, move the supporting member 105 for scanning in an appropriate scanning range, and perform measurement in a scanning time appropriate for the shape of the holder 103. The moving unit 111 may control the moving range in the Z-direction by controlling the distance between the supporting member 105 and the holder 103.

Specifically, the type of the holding cup 203 is recorded in the IC chip included in the holding cup 203, and two types of scanning ranges corresponding to two types of sizes are stored in the holder-shape determining unit 402. On the basis of information about the type of the holding cup 203 detected by the shape detecting unit 401, the holder-shape determining unit 402 determines which of the two types of scanning ranges is to be used. Thus, by controlling the moving unit 111, scanning is performed in the scanning range appropriate for the holding cup 203 placed.

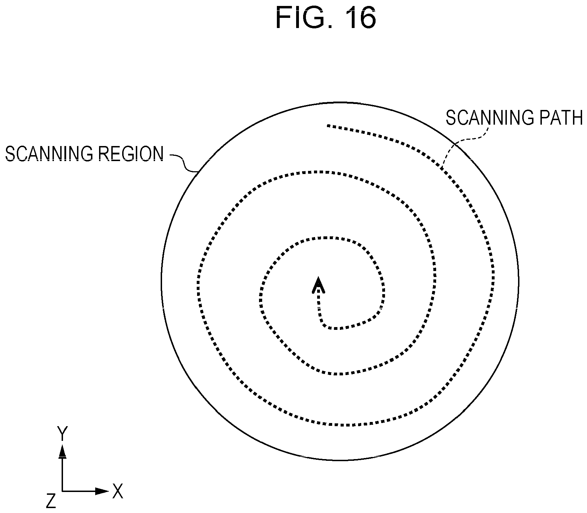

In the description above, the type of the holding cup 203 is recorded in the IC chip included in the holding cup 203. Alternatively, information about a specific scanning range may be recorded in the IC chip so that the holder-shape determining unit 402 controls the moving unit 111 on the basis of this information. Cup shape information, such as a cup depth and a curvature, may be recorded in the IC chip included in the holding cup 203 so that the holder-shape determining unit 402 calculates the scanning range on the basis of this information. With this configuration, there is no need to prepare scanning range information corresponding to a plurality of types of holding cups 203 in the holder-shape determining unit 402 in advance. This is advantageous in that it is possible to freely increase the number of shapes of the holding cup 203. A plurality of scanning ranges may be stored in the moving unit 111 so that the holder-shape determining unit 402 selects a scanning range to be used. In the present example, the acoustic wave detectors 104 scan the holding cup 203 (i.e., the examined portion 101) by moving the supporting member 105, which supports the acoustic wave detectors 104, with respect to the holding cup 203. A scanning path for moving the supporting member 105 within the scanning range may be any path as long as scanning can be performed throughout the scanning range. When the holder 103 is cup-shaped as described above, the scanning may be performed along a scanning path similar to the contour of the holding cup 203 for better scanning efficiency. For example, scanning may be performed along a spiral path (see Fig. 16) in the XY-plane in Fig. 1. This is desirable not only in terms of scanning efficiency, but also in terms of the following when the supporting member 105 is filled with the acoustic matching liquid 106 as described with reference to Fig. 1. That is, if scanning is performed along a spiral path when the supporting member 105 is filled with the acoustic matching liquid 106, it is possible to reduce the amount of change in acceleration applied to the acoustic matching liquid 106 during scanning. This can suppress vibration of the acoustic matching liquid 106 to a low level.

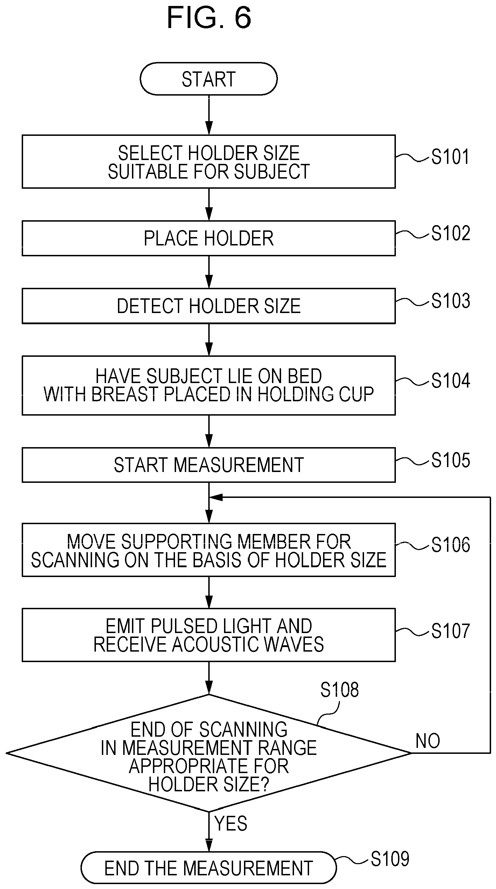

A flow of carrying out Example 3 will now be described with reference to Fig. 6.

In step S101, the size of the holding cup 203 that fits the shape of the examined portion 101 is selected. The holding cup 203 that covers the entire range of the examined portion 101 to be measured is selected. Next, the selected holding cup 203 is placed in the bed 102 (step S102).

The process proceeds to step S103, where the shape detecting unit 401 detects the shape of the holding cup 203. In step S104, the subject 100 lies face-down with the examined portion 101 placed in the holding cup 203.

After insertion of the examined portion 101 into the holding cup 203 is confirmed, the process proceeds to step S105, where the measurement starts.

In step S106, information about the shape of the holding cup 203 detected in step S103 is transmitted to the movement control unit 403. While the movement control unit 403 is controlling the scanning range, the light irradiation portion 110 emits pulsed light in step S107. Acoustic waves excited by the emitted pulsed light at a light absorber in the examined portion 101 are received by the acoustic wave detectors 104. A scanning range appropriate for the shape of the holding cup 203 is input to the movement control unit 403 in advance. The holding cup 203 is used as the holder 103 in the present example. When the sheet member 303 is used as the holder 103, the movement control unit 403 calculates a necessary scanning range from information about the shape measured by the shape detecting unit 401, such as a camera, and performs scanning.

In step S108, a determination is made as to whether the scanning has been completed. This is to check whether measurement has been performed for the entire scanning range set in advance.

If the scanning has been completed (YES in step S108), the process proceeds to step S109, where the measurement ends.

As described above, in the present example, the scanning is performed in a scanning range appropriate for the shape of the holder 103. This makes it possible to acquire a high-quality image of the range to be measured, and to perform measurement in a scanning time appropriate for the shape of the holder 103.

### (Example 4)

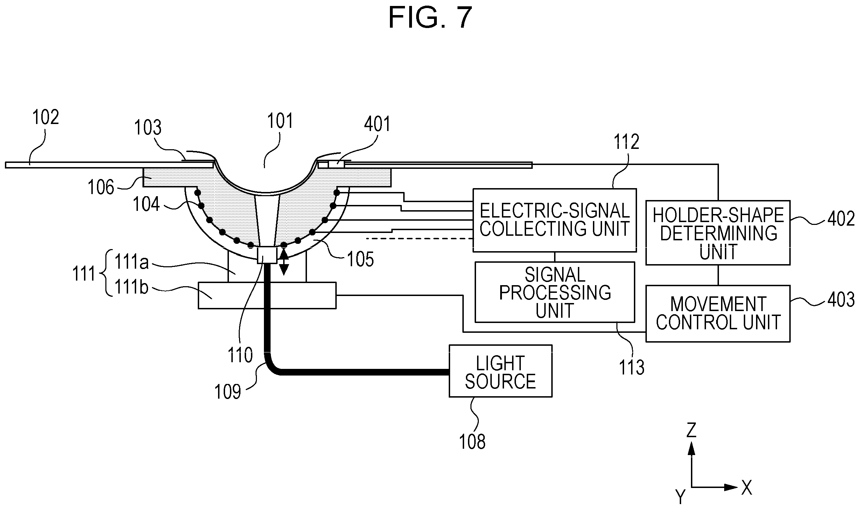

The intensity of pulsed light applied to the examined portion 101 varies (or changes) depending on the shape of the holder 103. In the present example, the position of the light irradiation portion 110 is controlled on the basis of the shape of the holder 103, so that the intensity of pulsed light applied to the examined portion 101 (irradiation intensity) is adjusted. Appropriate positional control of the light irradiation portion 110 based on the shape of the holder 103 will now be described with reference to Fig. 7. In the present example, the holding cup 203 is used as the holder 103. The components common to those in the examples described above are given the same reference numerals and their detailed description will be omitted.

In the present example, the light irradiation portion 110 is held by the supporting member 105 and is moved in the XYZ-directions as the supporting member 105 moves. Alternatively, the light irradiation portion 110 may be moved independently.

The sound pressure of acoustic wave signals is proportional to the amount of light that reaches an absorber. To increase the signal strength, it is necessary to increase the amount of light with which the examined portion 101 is irradiated.

When a laser is used as the light source 108, it is necessary to ensure that the maximum density of irradiation applied to a living body (i.e., the maximum amount of light for irradiation per unit area) does not exceed the maximum permissible exposure (MPE) defined in the laser safety standards (JIS C6802 and IEC 60825-1). Therefore, to increase the signal strength, it is necessary to maximize the amount of light for irradiation such that the MEP is not exceeded.

When the position of the light irradiation portion 110 with respect to the examined portion 101 changes, the irradiation density of light applied to the examined portion 101 is changed by diffusion of light. As the distance from the light irradiation portion 110 to the examined portion 101 increases, the irradiation density decreases. This attenuates the sound pressure of acoustic waves generated in the examined portion 101.

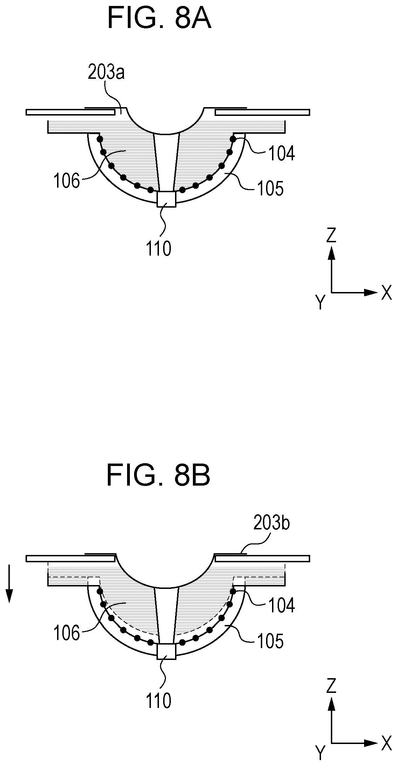

Fig. 8A illustrates how the small holding cup 203a is irradiated with light, and Fig. 8B illustrates how the large holding cup 203b is irradiated with light. If the large holding cup 203b is irradiated with light whose amount of emission from the light irradiation portion 110 (exit end) is regulated such that the irradiation density is appropriate for the examined portion 101 held by the small holding cup 203a, the MPE may be exceeded due to the short distance from the light irradiation portion 110 to the holder 103. Conversely, if the small holding cup 203a is irradiated with light whose amount of emission from the light irradiation portion 110 (exit end) is regulated such that the irradiation density is appropriate for the examined portion 101 held by the large holding cup 203b, since the irradiation density is low because of the long distance from the light irradiation portion 110 and the holder 103, the sound pressure of acoustic waves generated from the light absorber is weakened and the signal strength is reduced. To keep the amount of light reaching the holder 103 constant even when the shape of the holder 103 changes, it is necessary to control the position of the light irradiation portion 110 as illustrated in Figs. 8A and 8B. In the present example, an irradiation-intensity control unit configured to control the intensity of light for irradiating the holder 103 (i.e., the examined portion 101) controls the position of the light irradiation portion 110 on the basis of the shape of the holder 103. In the configuration of Fig. 7, the movement control unit 403 also serves as the irradiation-intensity control unit. That is, by controlling the moving unit 111 to control the distance between the holder 103 and the light irradiation portion 110 (light exit end) positioned in the supporting member 105, the movement control unit 403 controls the intensity of light for irradiating the examined portion 101.

In Example 3 described above, the scanning range appropriate for the cup size is recorded either in the holder-shape determining unit 402 or the IC chip in the holding cup 203. Similarly, in the present example, information about the position of the light irradiation portion 110 appropriate for the cup size may be recorded in the holder-shape determining unit 402 or the IC chip in the holding cup 203.

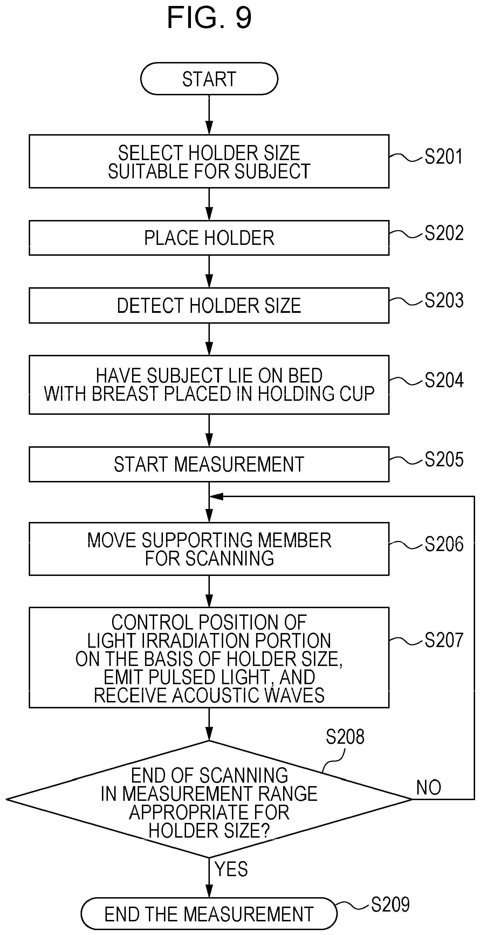

A flow of carrying out Example 4 will now be described with reference to Fig. 9. The description will center on the differences from Example 3.

Steps S201 to S205 in Fig. 9 are the same as steps S101 to S105 in Fig. 6.

While the supporting member 105 is being moved for scanning in the range to be measured (step S206), information about the shape of the holding cup 203 detected in step S203 is transmitted to the movement control unit 403, which controls the position of the light irradiation portion 110. Then the light irradiation portion 110 emits pulsed light, and acoustic waves excited by the emitted pulsed light at the light absorber in the examined portion 101 are received by the acoustic wave detectors 104 (step S207). Information about the position of the light irradiation portion 110 appropriate for the shape of the holding cup 203 is input to the movement control unit 403 in advance. The holding cup 203 is used as the holder 103 in the present example. When the sheet member 303 is used as the holder 103, irradiation is performed by calculating the appropriate position of the light irradiation portion 110 from information about the shape of the sheet member 303 measured by the shape detecting unit 401, such as a camera.

Steps S208 and S209 in Fig. 9 are the same as steps S108 and S109 in Fig. 6.

In the present example, as described above, an appropriate signal strength can be obtained by controlling the position of the light irradiation portion 110 on the basis of the shape of the holder 103.

### (Example 5)

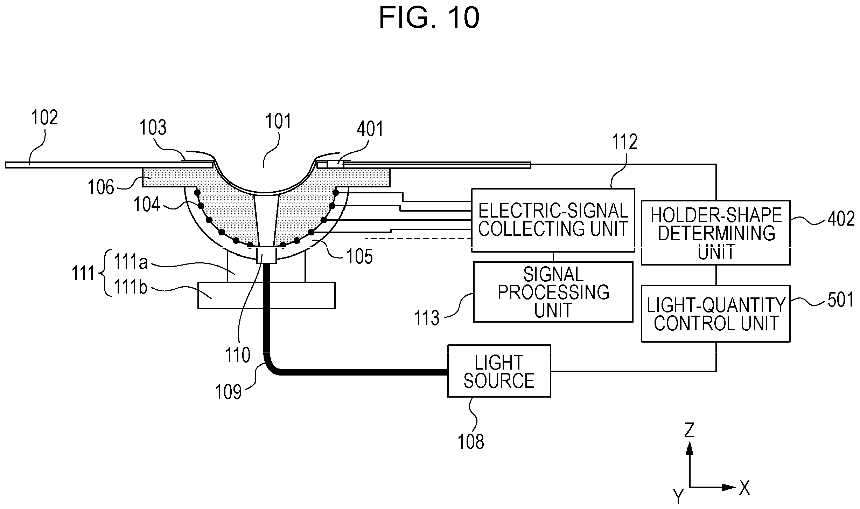

Fig. 10 illustrates a configuration of Example 5. In the present example, the holding cup 203 is used as the holder 103. The components common to those in the examples described above are given the same reference numerals and their detailed description will be omitted.

A light-quantity control unit 501 serves as an irradiation-intensity control unit that controls the amount of pulsed light generated from the light source 108. The light-quantity control unit 501 stores information about the amount of light appropriate for the shape of the holder 103 in advance.

In Examples 3 and 4 described above, the movement of the supporting member 105 is controlled by transmitting information about the shape of the holder 103 from the holder-shape determining unit 402 to the movement control unit 403. In Example 5, the amount of pulsed light generated from the light source 108 is controlled on the basis of information about the shape of the holder 103. The control method will now be described.

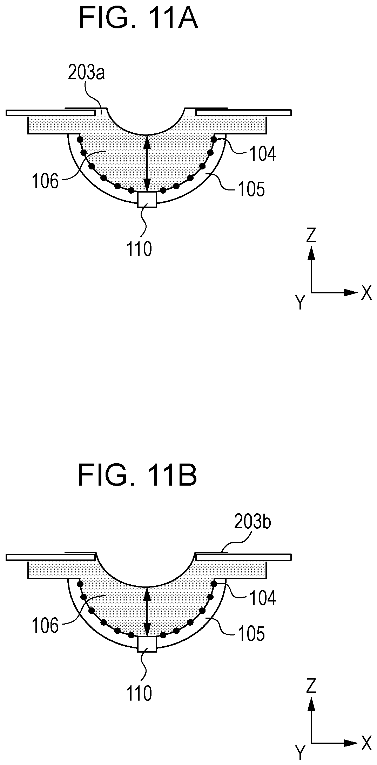

Fig. 11A illustrates a positional relationship between the small holding cup 203a and the light irradiation portion 110 serving as an exit end, and Fig. 11B illustrates a positional relationship between the large holding cup 203b and the light irradiation portion 110 serving as an exit end. When the position of the light irradiation portion 110 in the Z-direction is kept unchanged, the distance from the light irradiation portion 110 to the holder 103 is longer in the small holding cup 203a than that in the large holding cup 203b. Therefore, when the small holding cup 203a and the large holding cup 203b are irradiated with the same amount of light from the light irradiation portion 110, the amount of light applied to the small holding cup 203a is smaller than that applied to the large holding cup 203b. If the amount of light emitted from the light irradiation portion 110 is set such that the irradiation density is appropriate for the large holding cup 203b, the irradiation density of light actually applied to the small holding cup 203a is reduced and thus the signal strength is reduced. Conversely, if the amount of light emitted from the light irradiation portion 110 is set such that the irradiation density is appropriate for the small holding cup 203a, the irradiation density of light actually applied to the large holding cup 203b is increased and may exceed the MPE. To keep the irradiation density of light applied to the holder 103 to an appropriate level even when the shape of the holder 103 changes, it is necessary to regulate the amount of light applied to the holder 103 in accordance with the shape of the holder 103. In the present example, the irradiation-intensity control unit configured to control the irradiation intensity of light applied to the holder 103 (i.e., the examined portion 101) controls the amount of light from the light source 108 on the basis of the shape of the holder 103 so as to appropriately control the amount of light emitted from the light irradiation portion 110, and thus controls the irradiation intensity of light applied to the examined portion 101.

In Example 3 described above, the scanning range appropriate for the cup size is recorded either in the holder-shape determining unit 402 or the IC chip in the holding cup 203. Similarly, in the present example, the information about the amount of light irradiation appropriate for the cup size may be recorded in the holder-shape determining unit 402 or the IC chip in the holding cup 203.

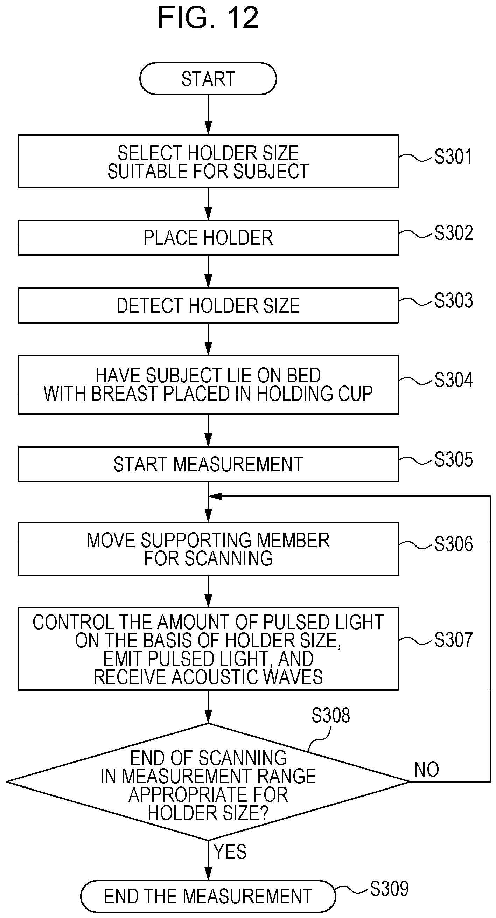

A flow of carrying out Example 5 will now be described with reference to Fig. 12. The description will center on the differences from Example 3.

Steps S301 to S305 in Fig. 12 are the same as steps S101 to S105 in Fig. 6.

While the supporting member 105 is being moved for scanning in the range to be measured (step S306), information about the shape of the holding cup 203 detected in step S303 is transmitted to the light-quantity control unit 501, so that information about the amount of light appropriate for the shape of the holding cup 203 is transmitted to the light source 108. Then the light irradiation portion 110 emits pulsed light, and acoustic waves excited by the emitted pulsed light at the light absorber in the examined portion 101 are received by the acoustic wave detectors 104 (step S307). The holding cup 203 is used as the holder 103 in the present example. When the sheet member 303 is used as the holder 103, the light-quantity control unit 501 calculates the appropriate amount of light from information about the shape of the sheet member 303 measured by the shape detecting unit 401, such as a camera, and controls the amount of light on the basis of the calculated data. Then irradiation is performed.

Steps S308 and S309 in Fig. 12 are the same as steps S108 and S109 in Fig. 6.

In the present example, as described above, a safe and appropriate signal strength can be obtained by controlling the amount of light on the basis of the shape of the holder 103.

### (Example 6)

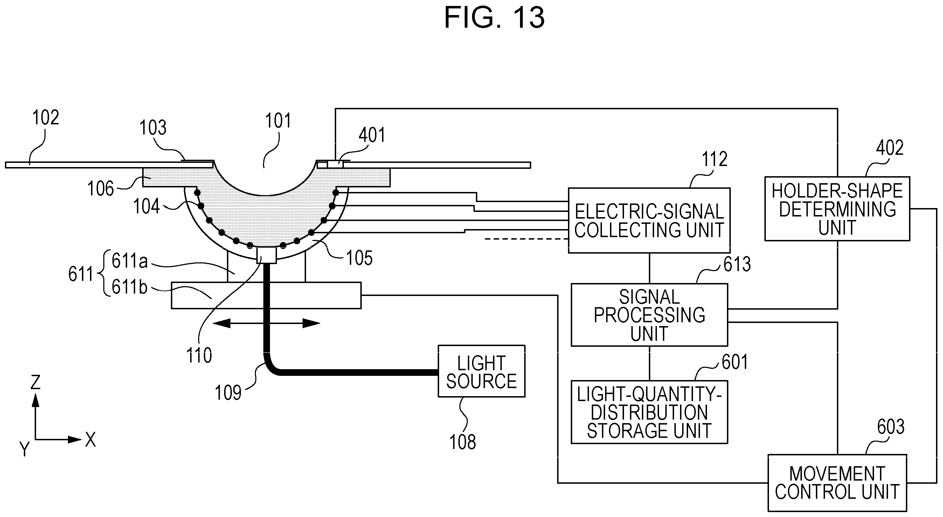

Example 6 will be described with reference to Fig. 13. The components common to those in the examples described above are given the same reference numerals and their detailed description will be omitted.

In the present example, the subject-information acquiring apparatus includes an estimating unit that estimates how the amount of light applied to the examined portion 101 is distributed in the examined portion 101 (light-quantity distribution information). The estimating unit estimates the distribution of the amount of light on the basis of the shape of the holder 103.

A light-quantity-distribution storage unit 601 in Fig. 13 stores light-quantity distribution information for the shape of each holder 103 and each position of the supporting member 105. A moving unit 611 that moves the supporting member 105 includes a Z-direction (vertical) moving mechanism 611a and an XY-direction (horizontal) moving mechanism 611b. Note that the Z-direction moving mechanism 611a is optional in the present example.

A movement control unit 603 controls the moving range of the moving unit 611 on the basis of the shape of the holder 103 determined by the holder-shape determining unit 402.

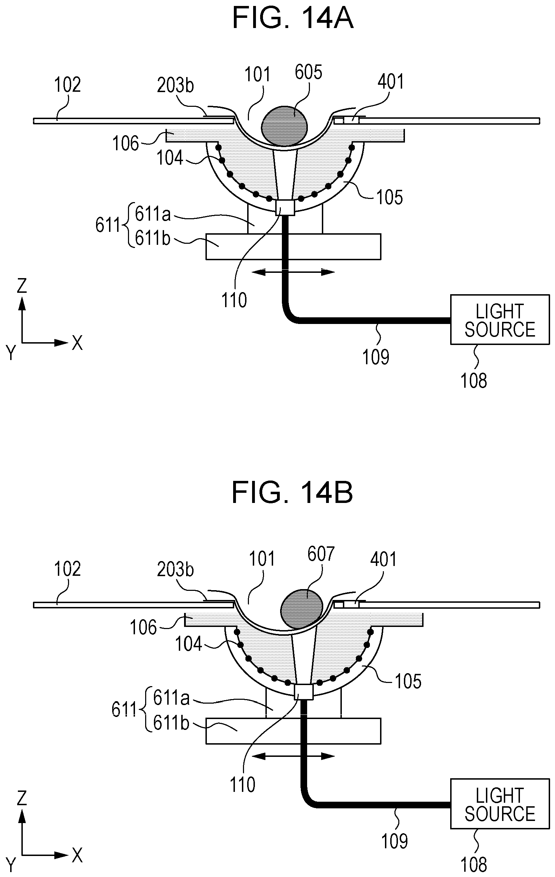

A signal processing unit 613 calculates the distribution of light absorption in the examined portion 101 from a plurality of electric signals and light distribution data, and acquires information about the distribution of initial sound pressure in the examined portion 101 with a technique, such as a delay-and-sum technique, from a plurality of electric signals. Additionally, on the basis of the shape of the holder 103 determined by the holder-shape determining unit 402 and the position of the supporting member 105 controlled by the movement control unit 603, the signal processing unit 613 refers to the light-quantity distribution information recorded in the light-quantity-distribution storage unit 601, and acquires information about the distribution of light absorption in the examined portion 101 by normalization through the use of the light-quantity distribution information. The light-quantity distribution information stored for each holder 103 in the light-quantity-distribution storage unit 601 will now be described with reference to Figs. 14A and 14B. As illustrated, the large holding cup 203b is used as the holder 103 here.

Fig. 14A illustrates the position of the supporting member 105 when a lowermost part of the large holding cup 203b is irradiated with light. Fig. 14B illustrates the position of the supporting member 105 when a peripheral part of the large holding cup 203b is irradiated with light. Both Figs. 14A and 14B schematically illustrate how the light reaches the examined portion 101. As illustrated, an illuminated region 605 formed by irradiating the lowermost part of the large holding cup 203b with light differs from an illuminated region 607 formed by irradiating the peripheral part of the large holding cup 203b with light. This is because since the holding cup 203 has a curvature, the area of irradiation on the holding cup 203 varies depending on the position of the supporting member 105 during irradiation. Even when the supporting member 105 is located at the same position, the illuminated region varies depending on the size of the holding cup 203. Therefore, it is desirable that the distribution of the amount of light be stored for each position of the supporting member 105 and the shape of each holder 103. In the present example, the distribution of the amount of light in the examined portion 101 having a three-dimensional shape is calculated for the shape of each holder 103 and each position of the supporting member 105 on the assumption that the examined portion 101 having a typical light absorption coefficient and a typical light scattering coefficient is held in the shape of the holder 103. The light-quantity distribution information is calculated in advance and stored in the light-quantity-distribution storage unit 601.

In the present example, information about the distribution of light absorption in the examined portion 101 can be acquired by taking into account the distribution of the amount of light corresponding to the shape of the holder 103. Therefore, it is possible to improve quantitativity of the light-quantity distribution information.

### (Example 7)

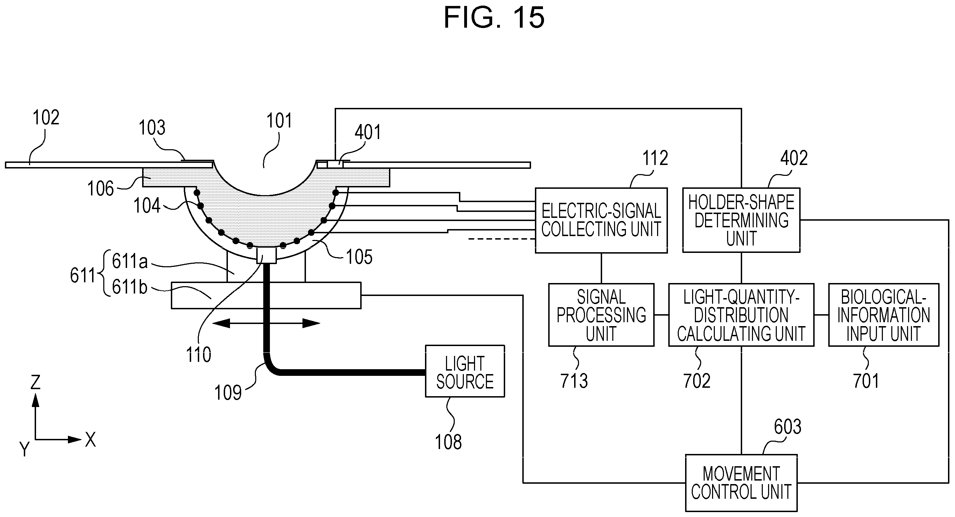

Example 7 will be described with reference to Fig. 15. The components common to those in the examples described above are given the same reference numerals and their detailed description will be omitted.

In the present example, the subject-information acquiring apparatus includes an estimating unit that estimates light-quantity distribution information by taking into account the light absorption coefficient and the light scattering coefficient of the examined portion 101.

The light absorption coefficient and the light scattering coefficient of the examined portion 101 measured in advance are input to a biological-information input unit 701 in Fig. 15. A light-quantity-distribution calculating unit 702 calculates the distribution of the amount of light in the examined portion 101 having a three-dimensional shape on the assumption that the examined portion 101 having the input light absorption coefficient and light scattering coefficient is held in the shape of the holder 103 (i.e., on the assumption that the contour of the examined portion 101 matches the shape of the holder 103). The light-quantity-distribution calculating unit 702 calculates the distribution of the amount of light in the examined portion 101 (light-quantity-distribution information) on the basis of the shape of the holder 103 determined by the holder-shape determining unit 402 and the position of the supporting member 105 controlled by the movement control unit 603. A signal processing unit 713 acquires information about the distribution of initial sound pressure in the examined portion 101 with a technique, such as a delay-and-sum technique, from a plurality of electric signals. Additionally, the signal processing unit 713 acquires information about the distribution of light absorption in the examined portion 101 by normalization through the use of the light-quantity distribution information calculated by the light-quantity-distribution calculating unit 702.

In the present example, the distribution of light absorption in the examined portion 101 can be acquired using the light-quantity distribution information obtained by taking into account the light absorption coefficient and the light scattering coefficient of the examined portion 101. Therefore, it is possible to further improve quantitativity of the light-absorption distribution information as compared to Example 6.

### (Example 8)

In Example 8, the subject-information acquiring apparatus includes an ultrasonic probe for generating an ultrasonic image of the examined portion 101 and controls the transmission focus of ultrasonic waves in accordance with the cup size and the position of the ultrasonic probe.

In the present example, ultrasonic data necessary for generating an intended three-dimensional ultrasonic image is obtained by repeating ultrasonic scanning while two-dimensionally moving the ultrasonic probe mounted on the moving unit 111. The term ultrasonic scanning refers to a process that involves electronically scanning the examined portion 101 with an ultrasonic beam generated by the ultrasonic probe and obtaining ultrasonic signals for generating B-mode tomographic image data.

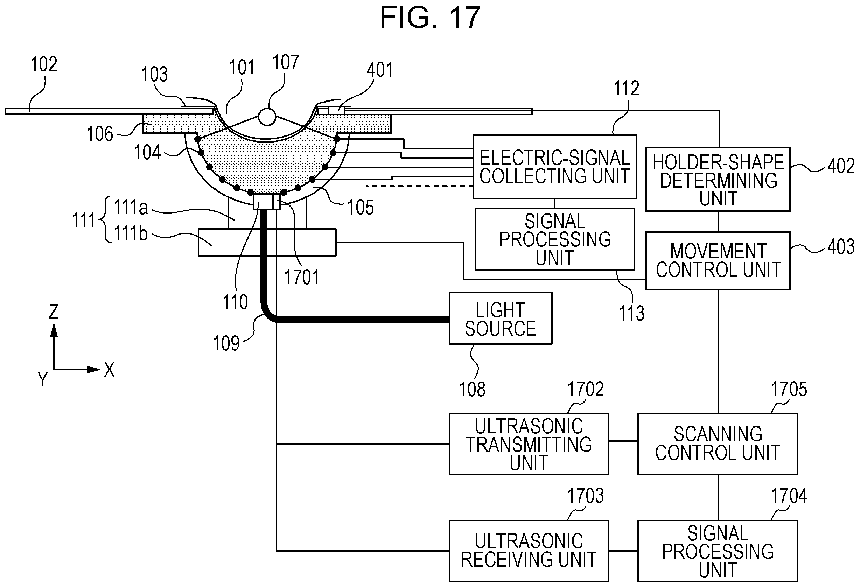

The present example will be described with reference to Fig. 17. Fig. 17 is a block diagram of the present example. The components common to those in the examples described above are given the same reference numerals and their detailed description will be omitted.

An ultrasonic probe 1701 transmits and receives ultrasonic waves. An ultrasonic transmitting unit 1702 applies drive signals to the ultrasonic probe 1701. An ultrasonic receiving unit 1703 amplifies signals detected by the ultrasonic probe 1701 and converts them into digital signals. A signal processing unit 1704 performs reception focus processing using detected ultrasonic signals. A scanning control unit 1705 controls the shape of an ultrasonic beam and the scanning with the ultrasonic beam. The moving unit 111 is equipped with an encoder (not shown). The movement control unit 403 obtains values from the encoder to determine the current position of the moving unit 111 or the ultrasonic probe 1701.

The ultrasonic probe 1701 includes a plurality of arranged acoustic elements. The ultrasonic probe 1701 transmits an ultrasonic beam to the examined portion 101, receives an ultrasonic echo reflected from the inside of the examined portion 101, and converts the received ultrasonic echo into electric signals. The ultrasonic probe 1701 used in the present example may be of any type. A transducer made of piezoelectric ceramics (PZT) or a microphone capacitive transducer used in typical ultrasonic diagnostic apparatuses is used as the ultrasonic probe 1701 (hereinafter, a probe for measurement of ultrasonic waves will be simply described as a probe). A capacitive micromachined ultrasonic transducer (CMUT), a magnetic MUT (MMUT) using a magnetic film, or a piezoelectric MUT (PMUT) using a piezoelectric thin film may also be used as the ultrasonic probe 1701.

In the present example, ultrasonic scanning performed using a one-dimensional ultrasonic probe which includes acoustic elements linearly arranged in a row is described for illustrative purposes. However, the application of the present invention is not limited to this. An array probe including acoustic elements two-dimensionally arranged (or 1.5D probe) may be used for ultrasonic scanning.

The scanning control unit 1705 generates drive signals to be applied to the respective acoustic elements of the ultrasonic probe 1701, and controls the frequency and sound pressure of ultrasonic waves to be transmitted. The scanning control unit 1705 has a transmission control function that sets an ultrasonic-beam transmitting direction and selects a transmission focus in accordance with the transmitting direction, and a reception control function that sets an ultrasonic-echo receiving direction and selects a reception focus in accordance with the receiving direction.

Transmission focusing is performed by setting a pattern of delay times given to a plurality of drive signals for forming an ultrasonic beam in a predetermined direction on the basis of ultrasonic waves transmitted from the acoustic elements. The details of how the transmission focus is determined will be described later on. A reception delay pattern is a pattern of delay times given to a plurality of received signals for extracting an ultrasonic echo from any direction on the basis of ultrasonic signals detected by the acoustic elements. The transmission focus and the reception delay pattern are stored in a storage medium (not shown).

The ultrasonic transmitting unit 1702 applies drive signals generated by the scanning control unit 1705 to the respective acoustic elements of the ultrasonic probe 1701.

The ultrasonic receiving unit 1703 includes a signal amplifier that amplifies analog signals detected by the acoustic elements of the ultrasonic probe 1701, and an analog-to-digital (A/D) converter that converts analog signals into digital signals. The ultrasonic receiving unit 1703 converts received signals into digital signals.

On the basis of the reception delay pattern selected by the scanning control unit 1705, the signal processing unit 1704 performs reception focus processing by adding signals corresponding to the delay times to the signals generated by the ultrasonic receiving unit 1703. This processing generates ultrasonic signals that converge to a particular focus.

By repeating ultrasonic scanning while two-dimensionally moving the ultrasonic probe 1701 mounted on the moving unit 111, ultrasonic data necessary for generating an intended three-dimensional ultrasonic image is obtained.

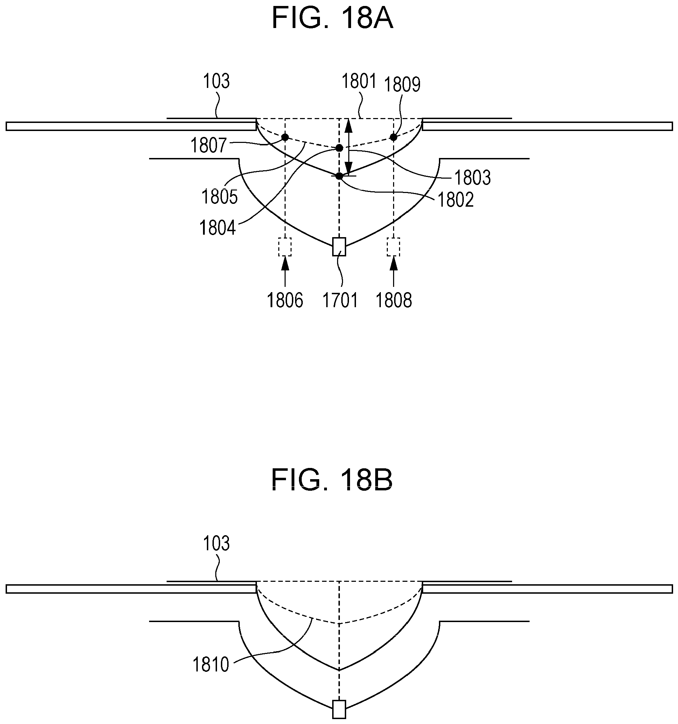

The relationship between the transmission focus, the cup size, and the position of the ultrasonic probe 1701 will now be described with reference to Figs. 18A and 18B. Fig. 18A illustrates the case where the cup size is small and the center of the ultrasonic probe 1701 is located at the center of the cup. Fig. 18B illustrates the case where the cup size is large. A deepest level (plane) 1801 of the breast is level with the holder 103. The deepest level 1801 represents the deepest point of measurement. An intersection point 1802 is a point at which a perpendicular line drawn from the ultrasonic probe 1701 to the deepest level 1801 of the measurement intersects the cup. A measurement distance 1803 is a distance from the intersection point 1802 of the perpendicular line and the cup to the deepest level 1801 of measurement. The measurement distance 1803 varies in accordance with the cup size and the position of the ultrasonic probe 1701. The measurement distance 1803 is calculated in advance from the cup size, the cup curvature, and the position of the ultrasonic probe 1701, and is stored in the storage medium (not shown). When the sheet member 303 is used as the holder 103, the measurement distance 1803 is calculated from the shape information measured by the shape detecting unit 401, such as a camera, and the position of the ultrasonic probe 1701, and is stored in the storage medium (not shown). A transmission focus position 1804 corresponds to the position of the ultrasonic probe 1701 illustrated in Fig. 18A. In the present example, the transmission focus position 1804 is at the midpoint of the measurement distance 1803. A transmission focus position 1805 is the position of the transmission focus for the small cup size. The transmission focus position 1805 varies in accordance with the position of the ultrasonic probe 1701. For example, a transmission focus position 1807 corresponds to a position 1806 of the ultrasonic probe 1701, and a transmission focus position 1809 corresponds to a position 1808 of the ultrasonic probe 1701. As can be seen, the transmission focus position 1805 changes as the measurement distance 1803 changes in accordance with the position of the ultrasonic probe 1701. A transmission focus position 1810 in Fig. 18B is the position of the transmission focus for the large cup size, and is calculated in the same manner as in Fig. 18A. The transmission focus position is calculated in advance from the cup size and the measurement distance, and is stored in the storage medium (not shown).

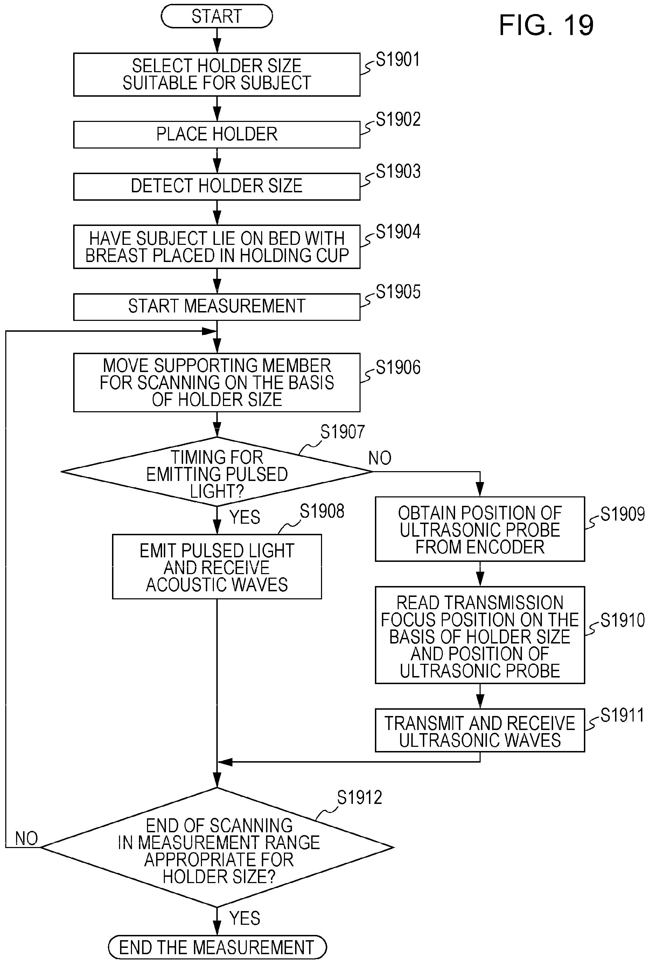

A flow of carrying out Example 8 will now be described with reference to Fig. 19.

Steps S1901 to S1906 in Fig. 19 are the same as steps S101 to S106 in Fig. 6 and their description will be omitted.

In the present example, the irradiation frequency of pulsed light from the light source 108 is 1 Hz.

In step S1907, a determination is made as to whether the timing for emitting the pulsed light is reached. If the timing for emission is reached, the process proceeds to step S1908, whereas if the timing for emission is not reached, the process proceeds to step S1909.

The description of step S1908 is omitted as it is the same as that of step S107 in Fig. 6.

In step S1909, the position of the ultrasonic probe 1701 is obtained from the encoder (not shown).

In step S1910, a transmission focus position is read from the storage medium (not shown) on the basis of the cup size detected in step S1903 and the position of the ultrasonic probe 1701 obtained in step S1909.

In step S1911, ultrasonic waves are transmitted and received on the basis of information about the transmission focus position read in step S1910.

The description of step S1912 is omitted as it is the same as that of step S108 in Fig. 6.

In the present example, ultrasonic waves are transmitted and received by controlling the transmission focus on the basis of the cup size or the shape information about the sheet member 303 and the position of the ultrasonic probe 1701. It is thus possible to obtain high-resolution ultrasonic data.

In the present invention, even when each examined portion 101 has a different shape, it is possible to change the shape of the holder 103 to fit the shape of the examined portion 101, and thus to acquire accurate information about the inside of the examined portion 101.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A subject-information acquiring apparatus comprising:
a light source (108);
a bed (102) having a holder (103) that holds an examined portion (101) which is part of a subject (100), the bed being configured to support the subject;
a plurality of acoustic wave detectors (104) configured to detect acoustic waves and outputs electric signals, the acoustic waves being generated when the examined portion held by the holder is irradiated with light emitted from the light source; and
signal processing means (113, 613, 713) configured to acquire information about the inside of the examined portion on the basis of the electric signals,
wherein the subject-information acquiring apparatus is capable of changing a shape of the holder so that the shape of the holder fits a shape of the examined portion,
wherein the subject-information acquiring apparatus further includes moving means (111) configured to move the plurality of acoustic wave detectors relative to the holder, and
wherein the moving means controls a moving range of the plurality of acoustic wave detectors based on the shape of the holder.

2. The subject-information acquiring apparatus according to Claim 1, wherein the holder is cup-shaped, and the holder is changed in shape to fit the shape of the examined portion by placing a cup-shaped member (203) having a size appropriate for the shape of the examined portion in the bed, the cup-shaped member being one of a plurality of cup-shaped members (203a, 203b) of different sizes.

3. The subject-information acquiring apparatus according to Claim 1, wherein the holder is an elastic member (303).

4. The subject-information acquiring apparatus according to Claim 3, wherein the moving means controls the moving range by controlling a distance between the acoustic wave detectors and the holder.

5. The subject-information acquiring apparatus according to Claim 3, wherein the moving means controls the moving range by controlling the amount of displacement of a center position of the acoustic wave detectors with respect to a center position of the holder.

6. The subject-information acquiring apparatus according to Claim 3, further comprising:
a light guiding portion (109) having an exit end (110) for emitting light from the light source to the holder; and
irradiation-intensity control means (403, 501) configured to control irradiation intensity of light with which the holder is irradiated, the light being emitted from the exit end,
wherein the irradiation-intensity control means controls the irradiation intensity on the basis of the shape of the holder.

7. The subject-information acquiring apparatus according to Claim 6, wherein the irradiation-intensity control means controls the irradiation intensity by controlling a distance between the exit end and the holder.

8. The subject-information acquiring apparatus according to Claim 6, wherein the irradiation-intensity control means controls the irradiation intensity by controlling the amount of light from the light source.

9. The subject-information acquiring apparatus according to Claim 1, further comprising estimating means configured to estimate light-quantity distribution information, the light-quantity distribution information being information about a distribution of the amount of light in the examined portion irradiated with the light,
wherein the signal processing means acquires information about the inside of the examined portion on the basis of the electric signals and the light-quantity distribution information; and
the estimating means estimates the distribution of the amount of light on the basis of the shape of the holder.

10. The subject-information acquiring apparatus according to claim 1 further comprising:
a supporting member configured to support the plurality of acoustic wave detectors such that a direction of maximum reception sensitivity of at least some of the plurality of acoustic wave detectors and a direction of maximum reception sensitivity of other acoustic wave detectors different from the at least some of the plurality of acoustic wave detectors are different and are directed toward a specific region,
wherein the moving means is configured to control the moving range by moving the supporting member.

## Patentansprüche

1. Vorrichtung zur Gewinnung von Objektinformationen, mit:
einer Lichtquelle (108);
einer Auflagefläche (102), die eine Halterung (103) aufweist, die einen untersuchten Bereich (101), der Teil eines Objekts (100) ist, aufnimmt, wobei die Auflagefläche eingerichtet ist, um das Objekt abzustützen;
einer Vielzahl von Schallwellenerfassungseinrichtungen (104), die dazu eingerichtet sind, um Schallwellen zu erfassen und elektrische Signale auszugeben, wobei die Schallwellen erzeugt werden, wenn der durch die Halterung aufgenommene untersuchte Bereich mit aus der Lichtquelle emittiertem Licht bestrahlt wird; und
einer Signalverarbeitungseinrichtung (113, 613, 713), die dazu eingerichtet ist, um basierend auf den elektrischen Signalen Informationen über das Innere des untersuchten Bereichs zu gewinnen,
wobei die Vorrichtung zur Gewinnung von Objektinformationen die Form der Halterung derart wechseln kann, dass die Form der Halterung zu einer Form des untersuchten Bereichs passt,
wobei die Vorrichtung zur Gewinnung von Objektinformationen des Weiteren eine Bewegungseinrichtung (111) aufweist, die dazu eingerichtet ist, um die Vielzahl von Schallwellenerfassungseinrichtungen relativ zur Halterung zu bewegen, und
wobei die Bewegungseinrichtung einen Bewegungsbereich der Vielzahl von Schallwellenerfassungseinrichtungen basierend auf der Form der Halterung steuert.

2. Vorrichtung zur Gewinnung von Objektinformationen gemäß Anspruch 1, wobei die Halterung becherförmig ist, und die Halterung, damit sie zu der Form des untersuchten Bereichs passt, in ihrer Form in der Auflagefläche geändert wird, indem ein becherförmiges Bauelement (203) platziert wird, das eine für die Form des untersuchten Bereichs angemessene Größe aufweist, wobei das becherförmige Bauelement ein Bauelement aus einer Vielzahl von becherförmigen Bauelementen (203a, 203b) unterschiedlicher Größe ist.

3. Vorrichtung zur Gewinnung von Objektinformationen gemäß Anspruch 1, wobei die Halterung ein elastisches Bauelement (303) ist.

4. Vorrichtung zur Gewinnung von Objektinformationen gemäß Anspruch 3, wobei die Bewegungseinrichtung den Bewegungsbereich durch das Steuern eines Abstands zwischen den Schallwellenerfassungseinrichtungen und der Halterung steuert.

5. Vorrichtung zur Gewinnung von Objektinformationen gemäß Anspruch 3, wobei die Bewegungseinrichtung den Bewegungsbereich durch das Steuern des Ausmaßes der Verschiebung einer Mittelstellung der Schallwellenerfassungseinrichtungen bezüglich einer Mittelstellung der Halterung steuert.

6. Vorrichtung zur Gewinnung von Objektinformationen gemäß Anspruch 3, des Weiteren mit:
einem lichtlenkenden Bereich (109), der ein Austrittsende (110) zur Emittierung von Licht aus der Lichtquelle auf die Halterung aufweist; und
einer Bestrahlungsintensitätssteuerungseinrichtung (403, 501), die dazu eingerichtet ist, um eine Bestrahlungsintensität des Lichts, mit dem die Halterung bestrahlt wird, zu steuern, wobei das Licht aus dem Austrittsende emittiert wird,
wobei die Bestrahlungsintensitätssteuerungseinrichtung die Bestrahlungsintensität basierend auf der Form der Halterung steuert.

7. Vorrichtung zur Gewinnung von Objektinformationen gemäß Anspruch 6, wobei die Bestrahlungsintensitätssteuerungseinrichtung die Bestrahlungsintensität durch das Steuern eines Abstands zwischen dem Austrittsende und der Halterung steuert.

8. Vorrichtung zur Gewinnung von Objektinformationen gemäß Anspruch 6, wobei die Bestrahlungsintensitätssteuerungseinrichtung die Bestrahlungsintensität durch das Steuern der Lichtmenge von der Lichtquelle steuert.

9. Vorrichtung zur Gewinnung von Objektinformationen gemäß Anspruch 1, des Weiteren mit einer Schätzeinrichtung, die dazu eingerichtet ist, um Lichtmengenverteilungsinformationen zu schätzen, wobei die Lichtmengenverteilungsinformationen Informationen über eine Verteilung der Lichtmenge in dem mit dem Licht bestrahlten untersuchten Bereich sind,
wobei die Signalverarbeitungseinrichtung Informationen über das Innere des untersuchten Bereichs, basierend auf den elektrischen Signalen und den Lichtmengenverteilungsinformationen, erfasst; und
die Schätzeinrichtung die Verteilung der Lichtmenge, basierend auf der Form der Halterung, schätzt.

10. Vorrichtung zur Gewinnung von Objektinformationen gemäß Anspruch 1, des Weiteren mit:
einem abstützenden Bauelement, das dazu eingerichtet ist, um die Vielzahl von Schallwellenerfassungseinrichtungen derart abzustützen, dass eine Richtung maximaler Empfangsempfindlichkeit von zumindest einigen der Vielzahl von Schallwellenerfassungseinrichtungen, und eine Richtung maximaler Empfangsempfindlichkeit anderer, sich von den zumindest einigen der Vielzahl von Schallwellenerfassungseinrichtungen unterscheidenden Schallwellenerfassungseinrichtungen unterschiedlich sind und zu einem spezifischen Bereich hingerichtet sind,
wobei die Bewegungseinrichtung dazu eingerichtet ist, um den Bewegungsbereich zu steuern, indem das abstützende Bauelement bewegt wird.

## Revendications

1. Appareil d'acquisition d'informations sur un sujet, comprenant :
une source de lumière (108) ;
un bâti (102) présentant un support (103) qui porte une partie examinée (101) qui fait partie d'un sujet (100), le bâti étant configuré pour porter le sujet ;
une pluralité de détecteurs d'ondes acoustiques (104) configurés pour détecter les ondes acoustiques et émettre des signaux électriques, les ondes acoustiques étant générées lorsque la partie examinée portée par le support est irradiée par une lumière émise par la source de lumière ; et
des moyens de traitement de signal (113, 613, 713) configurés pour acquérir des informations concernant l'intérieur de la partie examinée sur la base des signaux électriques,
dans lequel l'appareil d'acquisition d'informations sur un sujet est apte à modifier une forme du support de sorte que la forme du support s'adapte à une forme de la partie examinée,
dans lequel l'appareil d'acquisition d'informations sur un sujet comporte en outre des moyens de déplacement (111) configurés pour déplacer la pluralité de détecteurs d'ondes acoustiques par rapport au support, et
dans lequel les moyens de déplacement commandent une plage de déplacement de la pluralité de détecteurs d'ondes acoustiques sur la base de la forme du support.

2. Appareil d'acquisition d'informations sur un sujet selon la revendication 1, dans lequel le support est en forme de gobelet, et la forme du support est modifiée de manière à s'adapter à la forme de la partie examinée en plaçant un élément en forme de gobelet (203) présentant une taille appropriée à la forme de la partie examinée dans le bâti,
l'élément en forme de gobelet étant un parmi une pluralité d'éléments en forme de gobelet (203a, 203b) de différentes tailles.

3. Appareil d'acquisition d'informations sur un sujet selon la revendication 1, dans lequel le support est un élément élastique (303).

4. Appareil d'acquisition d'informations sur un sujet selon la revendication 3, dans lequel les moyens de déplacement commandent la plage de déplacement en commandant une distance entre les détecteurs d'ondes acoustiques et le support.

5. Appareil d'acquisition d'informations sur un sujet selon la revendication 3, dans lequel les moyens de déplacement commandent la plage de déplacement en commandant la quantité de déplacement d'une position centrale des détecteurs d'ondes acoustiques par rapport à une position centrale du support.

6. Appareil d'acquisition d'informations sur un sujet selon la revendication 3, comprenant en outre :
une partie de guidage de lumière (109) présentant une extrémité de sortie (110) destinée à émettre de la lumière depuis la source de lumière vers le support ; et
des moyens de commande d'intensité d'irradiation (403, 501) configurés pour commander l'intensité d'irradiation de la lumière au moyen de laquelle le support est irradié, la lumière étant émise à partir de l'extrémité de sortie,
dans lequel les moyens de commande d'intensité d'irradiation commandent l'intensité d'irradiation sur la base de la forme du support.

7. Appareil d'acquisition d'informations sur un sujet selon la revendication 6, dans lequel les moyens de commande d'intensité d'irradiation commandent l'intensité d'irradiation en commandant une distance entre l'extrémité de sortie et le support.

8. Appareil d'acquisition d'informations sur un sujet selon la revendication 6, dans lequel les moyens de commande d'intensité d'irradiation commandent l'intensité d'irradiation en commandant la quantité de lumière provenant de la source de lumière.

9. Appareil d'acquisition d'informations sur un sujet selon la revendication 1, comprenant en outre des moyens d'estimation configurés pour estimer les informations de distribution de quantité de lumière, les informations de distribution de quantité de lumière étant des informations concernant une distribution de la quantité de lumière dans la partie examinée irradiée par la lumière,
dans lequel les moyens de traitement de signal acquièrent des informations concernant l'intérieur de la partie examinée sur la base des signaux électriques et des informations de distribution de quantité de lumière ; et
les moyens d'estimation estiment la distribution de la quantité de lumière sur la base de la forme du support.

10. Appareil d'acquisition d'informations sur un sujet selon la revendication 1, comprenant en outre :
un élément de support configuré pour porter la pluralité de détecteurs d'ondes acoustiques de telle sorte qu'une direction de sensibilité de réception maximale d'au moins certains de la pluralité de détecteurs d'ondes acoustiques et une direction de sensibilité de réception maximale d'autres détecteurs d'ondes acoustiques différents des au moins certains de la pluralité de détecteurs d'ondes acoustiques sont différentes et sont dirigées vers une zone spécifique,
dans lequel les moyens de déplacement sont configurés pour commander la plage de déplacement en déplaçant l'élément de support.
